# FASCICULE DE BREVET EUROPEEN

(11) **EP 3 668 965 B1**
(45) Date de publication et mention de la délivrance du brevet: **23.03.2022**
(21) Numéro de dépôt: 18769755.2
(22) Date de dépôt: 09.08.2018
(51) Int. Cl.: C12M 1/107, C12M 1/12

(54) **RÉACTEUR DE MÉTHANATION BIOLOGIQUE**
BIOLOGISCHER METHANISIERUNGSREAKTOR
BIOLOGICAL METHANATION REACTOR

(30) Priorité: 16.08.2017 FR 1757703
(43) Date de publication de la demande: 24.06.2020
(73) Titulaire: ENGIE, 92400 Courbevoie (FR)
(72) Inventeur: FORTIN, Stéphane, 59850 Nieppe (FR); KARA, Yilmaz, 95600 Eaubonne (FR); CAPELA, Sandra, 92210 Saint-Cloud (FR); MAHEUT, Marion, 75005 Paris (FR)
(74) Mandataire: Cornuejols, Georges
(86) Numéro de dépôt international: PCT/FR2018/052041
(87) Numéro de publication internationale: WO 2019/034820

(56) Documents cités:
- EP-A2- 1 574 581
- WO-A2-2011/073618
- US-A- 4 322 296
- US-A- 4 571 384

## Description

### DOMAINE TECHNIQUE DE L'INVENTION

La présente invention vise un réacteur de méthanation biologique. Elle s'applique, notamment, au domaine de la méthanation industrielle pour produire un gaz riche en méthane de synthèse par conversion de dioxyde de carbone et de dihydrogène.

### ÉTAT DE LA TECHNIQUE

Les technologies de méthanation biologique sont parfois utilisées pour augmenter la teneur en méthane des biogaz issus de méthanisation biologique. La méthanisation biologique produit un biogaz riche en méthane et en dioxyde de carbone et contenant un certain nombre de composés minoritaires issus de la fermentation tels que l'ammoniac, l'hydrogène sulfuré, les siloxanes et autres. Les proportions entre méthane et dioxyde de carbone varient d'une méthanisation à une autre, mais le ratio 50/50 donne un ordre de grandeur des quantités relatives de ces deux constituants majoritaires. La production de dioxyde de carbone lors de la méthanisation est inévitable, mais elle représente une partie significative du carbone introduit initialement dans le méthaniseur non valorisé en méthane et c'est de plus un composé qu'il faut éliminer si l'on souhaite injecter le méthane dans le réseau de gaz naturel.

Pour éviter ce double inconvénient, il est possible de méthaner le dioxyde de carbone du biogaz par le biais de deux techniques :
- la méthanation thermochimique et
- la méthanation biologique.

Dans les deux cas, pour réaliser la méthanation on mélange du dioxyde de carbone et du dihydrogène dans un réacteur.

Dans la méthanation thermochimique, la réaction s'effectue en phase gazeuse à haute température (à une température d'environ 300 à 400°C) sous pression plus ou moins importante et en présence d'un catalyseur.

Dans la méthanation biologique, la réaction s'effectue en phase liquide grâce à des microorganismes méthanogènes du domaine Archaea par exemple.

Dans les deux cas, la réaction globale de méthanation s'écrit :

CO₂ + 4 H₂ → CH₄ + 2 H₂O + Chaleur

Comme de nombreux processus biologiques plusieurs conditions doivent être réunies pour que la réaction se déroule correctement, parmi celles-ci on peut noter :
- un milieu anaérobique humide,
- une température adéquate,
- la présence de nutriments et
- l'accès aux réactifs des microorganismes.

Si la culture en milieu anaérobique humide dans un domaine de température satisfaisant et la présence des nutriments peuvent facilement être gérées, l'accès aux réactifs que sont le dihydrogène et le dioxyde de carbone peut poser des problèmes car le dihydrogène est peu soluble dans l'eau et nettement moins soluble que le dioxyde de carbone. Or la réaction de méthanation nécessite un ratio H₂/CO₂ théorique de 4 / 1, le non-respect de cette stoechiométrie, ou la difficulté d'accès aux réactifs induisent des pertes d'efficacité, l'excès de réactifs dans le gaz produit et par conséquent le risque que celui-ci ne soit pas conforme aux spécifications d'injection sur un réseau de gaz naturel et qu'un traitement coûteux doit être mis en oeuvre pour éliminer ces excès.

Plusieurs travaux ont été réalisés pour tester la possibilité de réaliser la méthanation biologique en laboratoire et au stade industriel. Parmi ceux-ci, on peut citer :
- le lit bactérien à percolation et
- le réacteur agité.

Le lit bactérien est un réacteur qui utilise un matériau support pour le développement des microorganismes et ce matériau est aspergé d'eau pour maintenir le milieu humide et pour permettre le transfert des réactifs gazeux dans l'eau afin que les microorganismes y accèdent. Le gaz réactif est introduit par la base du réacteur.

Le lit bactérien (tel que décrit dans le document DE 10 2011 051 836) se caractérise par une circulation à contre-courant des flux liquide et gazeux. Le gaz tend à circuler selon un modèle dit piston ce qui favorise le rendement de la réaction. Cependant la circulation peut être entravée par la croissance bactérienne sur les supports et induire des passages préférentiels. Pour limiter ce type de problème le débit de gaz est relativement faible pour laisser un temps de contact suffisant entre les réactifs et les microorganismes immobilisées sur le support. Le lit bactérien se caractérise par des taux de production relativement faible (1.17 Nm³ CH₄/m³/jour) et donc une emprise au sol et un encombrement volumique important. Cet encombrement est lié au fait qu'il est nécessaire de maintenir un volume vide important pour laisser des passages libres au gaz et au liquide et de ne pas engorger le matériau support.

Le réacteur agité est un réacteur équipé d'un agitateur tournant à grande vitesse pour disperser dans le milieu les gaz et notamment l'hydrogène en fines bulles ainsi que les microorganismes afin d'augmenter l'accès des microorganismes aux réactifs.

Le réacteur agité a de bons taux de production : des valeurs de 100 à 200 Nm³ CH₄/m³/jour par exemple. Mais il nécessite une traversée de paroi (pour l'agitateur), ce qui pose des problèmes relatifs à l'étanchéité du système, et donc à la sécurité, de plus l'agitateur induit une consommation d'énergie. De par sa conception, ce type de réacteur est dit de type parfaitement agité, donc la distribution des temps de séjour y est très étalée : du très court au très long ; aussi une fuite de réactif gazeux plus ou moins importante a toujours lieu. Enfin dans ce type de réacteur les microorganismes sont en cultures dite libre et par conséquent elles sont très diluées dans le milieu, ce qui ne favorise pas le contact entre les microorganismes et les réactifs. Ce problème est exacerbé par le fait que la méthanation biologique produit de l'eau qui vient diluer le milieu et qu'il faut évacuer car le réacteur reste une enceinte à volume fini US4571384 divulgue une méthode pour la production de méthane à partir de dioxyde de carbone et de dihydrogène. La méthode emploi une enceinte avec un matériau de support pour des microorganismes producteurs de méthane et constitué de particules de carbonate minéral.

Aucun de ces enseignements ne permet d'optimiser l'accès des microorganismes aux réactifs pour respecter au mieux la stoechiométrie préférentielle de la réaction de méthanation tout en ayant un réacteur compact.

### OBJET DE L'INVENTION

La présente invention vise à remédier à tout ou partie de ces inconvénients.

À cet effet, la présente invention vise un réacteur de méthanation biologique de dihydrogène ou de gaz riche en dihydrogène et de dioxyde de carbone ou de gaz riche en dioxyde de carbone, qui comporte :
- une enceinte présentant une extrémité longitudinale dite « basse » et une extrémité longitudinale opposée dite « haute », ladite enceinte comportant, à proximité de l'extrémité basse :
   - une entrée primaire d'eau,
   - une entrée de dihydrogène ou du gaz riche en dihydrogène et
   - une entrée de dioxyde de carbone ou du gaz riche en dioxyde de carbone et à proximité de l'extrémité haute :
   - une sortie pour méthane de synthèse ou pour gaz riche en méthane de synthèse et
   - une sortie primaire pour eau,
- un matériau support pour former un lit de flore méthanogène, présentant une densité inférieure à la densité de l'eau, mobile en translation le long de l'axe longitudinal de l'enceinte, configuré pour recevoir une flore méthanogène et
- entre le matériau support d'une part et la sortie pour méthane et la sortie pour eau d'autre part, une surface de rétention du matériau support, perforée, formant butée pour le déplacement longitudinal du matériau support au niveau de la position de ladite surface.

Grâce à ces dispositions, l'accès des réactifs à la flore méthanogène est amélioré car cette flore est concentrée sur le matériau support. Le support mobile sert de site de colonisation aux microorganismes et de par son ratio surface/volume il permet de créer une grande surface de contact entre les microorganismes et le milieu. De plus, en immobilisant les microorganismes sur un support, on obtient un effet de concentration de la biomasse plus important que dans le cas des cultures libres. Ces deux effets concourent à augmenter la capacité réactionnelle par unité de volume et donc à réduire l'empreinte au sol du réacteur. Ils permettent aussi d'obtenir des résiduels très faibles en réactifs dans le gaz produit.

Dans des modes de réalisation, le réacteur objet de la présente invention comporte une surverse de collecte d'eau positionnée en amont de la sortie pour méthane ou pour gaz riche en méthane et de la sortie pour eau et en aval de la surface de rétention le long de l'axe de déplacement longitudinal du matériau support dans l'enceinte, orientée vers l'extrémité haute de l'enceinte, formant collecteur pour l'eau franchissant la surverse, la sortie pour eau étant positionnée dans ce collecteur.

Ces modes de récupération permettent la récupération d'eau dépourvue au maximum de méthane de synthèse. De plus, ces modes de réalisation permettent de limiter le déplacement du matériau support dans l'enceinte en formant une butée haute.

Dans des modes de réalisation, le réacteur objet de la présente invention comporte :
- au moins une pompe de recirculation de l'eau traversant la sortie pour eau de l'enceinte et
- au moins un échangeur thermique configuré pour chauffer ou refroidir l'eau sortie de l'enceinte,
l'eau recirculée, en sortie de l'échangeur thermique, étant au moins partiellement réinjectée dans l'enceinte à travers une conduite d'injection.

Ces modes de réalisation permettent de refroidir ou de réchauffer le milieu à l'intérieur de l'enceinte. À l'état initial du réacteur, un échauffement de ce milieu peut être favorable pour favoriser le développement de la flore méthanogène tandis qu'en opération, étant donné le caractère exothermique de la réaction de méthanation, il peut être favorable de réduire la température à l'intérieur de l'enceinte.

Dans des modes de réalisation, le réacteur objet de la présente invention comporte un capteur de mesure du niveau d'eau dans l'enceinte et une évacuation d'eau du réacteur, l'ouverture de l'évacuation étant commandée en fonction du niveau d'eau capté.

Dans des modes de réalisation, le capteur de mesure du niveau dans l'enceinte commande l'ouverture d'une entrée d'eau lorsque le niveau d'eau capté est inférieur à valeur limite prédéterminée.

Ces modes de réalisation permettent d'évacuer le surplus d'eau du réacteur ou l'ajout d'eau si nécessaire.

Dans des modes de réalisation, l'eau recirculée est injectée dans un flux comportant au moins du dihydrogène et/ou du dioxyde de carbone, en amont de l'entrée pour dihydrogène et/ou de l'entrée pour dioxyde de carbone dans l'enceinte.

Ces modes de réalisation permettent de favoriser la miscibilité des réactifs dans l'eau, en particulier lorsque la température de l'eau a été abaissée car la miscibilité des gaz dans l'eau est améliorée à basse température.

Dans des modes de réalisation, l'enceinte comporte une entrée pour nutriments et/ou réactifs additionnels et/ou d'eau.

Ces modes de réalisation permettent d'apporter au milieu à l'intérieur de l'enceinte de quoi favoriser le développement de la flore méthanogène ou de quoi éviter des effets auxiliaires de la réaction susceptibles de perturber le procédé, tel la formation de mousse par exemple.

Dans des modes de réalisation, les nutriments et/ou réactifs additionnels sont injectés dans la conduite d'injection en eau recirculée.

Ces modes de réalisation permettent de limiter le nombre d'ouvertures dans l'enceinte de manière à en favoriser l'étanchéité.

Dans des modes de réalisation, le réacteur objet de la présente invention comporte, à proximité de l'extrémité haute de l'enceinte, une entrée secondaire pour eau et, à proximité de l'extrémité basse de l'enceinte, une sortie secondaire de reprise d'eau.

Ces modes de réalisation permettent de laver l'intérieur de l'enceinte en y injectant de l'eau en haut et en collectant l'eau en bas, ainsi que les résidus étant détachés de l'intérieur de l'enceinte et du matériau support. Le cas échéant ces entrée et sortie d'eau permettent de réguler le niveau d'eau dans le réacteur en association avec le moyen de mesure du niveau d'eau dans le réacteur.

Dans des modes de réalisation, le réacteur objet de la présente invention comporte une unité de commande configurée pour commander l'actionnement d'un mode de fonctionnement de réacteur parmi au moins deux, dans lequel :
- dans un mode actif :
   - l'entrée secondaire pour eau et la sortie secondaire de reprise d'eau sont désactivées et
   - l'entrée primaire d'eau, l'entrée de dihydrogène, l'entrée de dioxyde de carbone et la sortie primaire d'eau sont activées et
- dans un mode de lavage :
   - l'entrée primaire d'eau, l'entrée de dihydrogène, l'entrée de dioxyde de carbone et la sortie primaire d'eau sont désactivées et
   - l'entrée secondaire pour eau et la sortie secondaire de reprise d'eau sont activées.

Dans des modes de réalisation, le réacteur objet de la présente invention comporte un moyen de mesure de perte de charge entre l'entrée et la sortie du réacteur représentative de la perte de charge dans le lit support, un changement de mode de fonctionnement étant commandé en fonction de la perte de charge mesurée.

Ces modes de réalisation permettent d'optimiser le déclenchement du mode de lavage.

Dans des modes de réalisation, le réacteur comporte, en partie basse, un plancher perforé et un espace libre entre le bas du matériau support et le plancher suffisamment important pour qu'en mode de lavage le matériau support subissant une expansion ou une fluidisation liée à l'écoulement ne soit pas entrainé par le débit d'eau dans le système de reprise d'eau.

Dans des modes de réalisation, le système de reprise est dimensionné pour empêcher l'entrainement du matériau support lors du lavage, soit en utilisant des crépines ou une grille ou tout autre dispositif permettant de retenir le matériau support lors de la phase de lavage, soit par le dimensionnement de l'espace libre et le débit d'eau de lavage.

Dans des modes de réalisation, l'entrée pour dihydrogène ou gaz riche en dihydrogène, l'entrée pour dioxyde de carbone ou gaz riche en dioxyde de carbone et l'entrée primaire d'eau sont confondues.

Dans des modes de réalisation, le réacteur objet de la présente invention comporte, en amont de l'entrée confondue d'eau, de dihydrogène et de dioxyde de carbone, un moyen de dissolution du dioxyde de carbone ou du gaz riche en dioxyde de carbone et du dihydrogène ou du gaz riche en dihydrogène dans l'eau.

Ces modes de réalisation permettent d'optimiser la dissolution du dioxyde de carbone et du dihydrogène dans l'eau en amont de l'enceinte.

Dans des modes de réalisation, l'eau recirculée est injectée au moins en partie dans le moyen de dissolution.

Dans des modes de réalisation, le réacteur objet de la présente invention comporte un moyen de dissolution du dioxyde de carbone ou du gaz riche en dioxyde de carbone dans l'eau d'une part et un moyen de dissolution du dihydrogène ou du gaz riche en dihydrogène dans l'eau d'autre part, chaque moyen de dissolution étant alimenté en eau par de l'eau recirculée, le débit de recirculation d'eau vers chaque moyen de dissolution étant commandé indépendamment.

Ces modes de réalisation permettent de faire varier la stœchiométrie de réactifs de méthanation à l'intérieur de l'enceinte grâce à la recirculation d'eau.

### BRÈVE DESCRIPTION DES FIGURES

D'autres avantages, buts et caractéristiques particulières de l'invention ressortiront de la description non limitative qui suit d'au moins un mode de réalisation particulier du dispositif objet de la présente invention, en regard des dessins annexés, dans lesquels :
- la figure 1 représente, schématiquement, un premier mode de réalisation particulier du dispositif objet de la présente invention,
- la figure 2 représente, schématiquement, un deuxième mode de réalisation particulier du dispositif objet de la présente invention,
- la figure 3 représente, schématiquement, une troisième mode de réalisation particulier du dispositif objet de la présente invention,
- la figure 4 représente, schématiquement, un quatrième mode de réalisation particulier du dispositif objet de la présente invention,
- la figure 5 représente, schématiquement, un cinquième mode de réalisation particulier du dispositif objet de la présente invention et
- la figure 6 représente, schématiquement, un sixième mode de réalisation particulier du dispositif objet de la présente invention.

### DESCRIPTION D'EXEMPLES DE RÉALISATION DE L'INVENTION

La présente description est donnée à titre non limitatif, chaque caractéristique d'un mode de réalisation pouvant être combinée à toute autre caractéristique de tout autre mode de réalisation de manière avantageuse.

On note dès à présent que les figures ne sont pas à l'échelle.

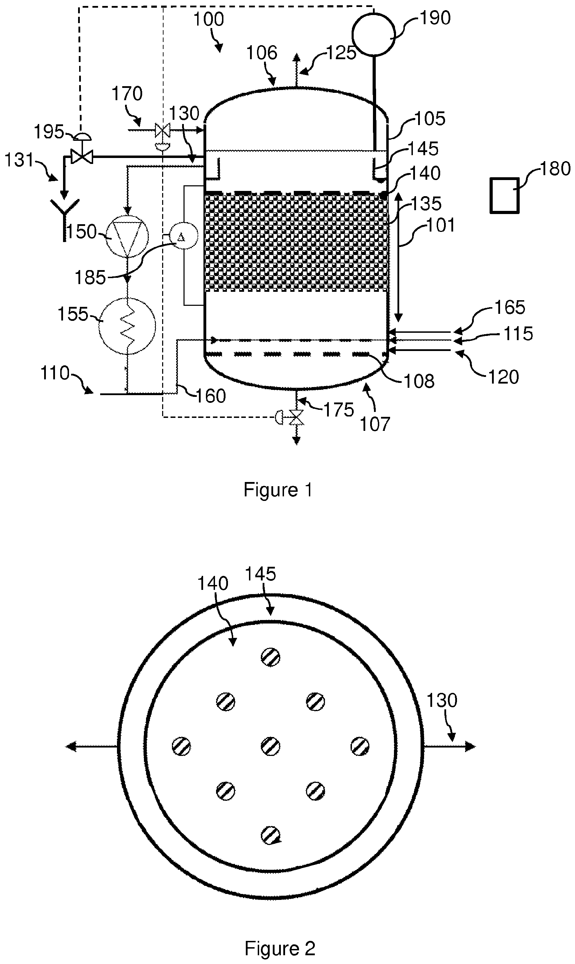

On observe, sur la figure 1, qui n'est pas à l'échelle, une vue schématique d'un mode de réalisation du réacteur 100 objet de la présente invention. Ce réacteur 100 de méthanation biologique de dihydrogène ou d'un gaz riche en dihydrogène et de dioxyde de carbone ou d'un gaz riche en dioxyde de carbone, comporte :
- une enceinte 105 présentant une extrémité 107 longitudinale dite « basse » et une extrémité 106 longitudinale opposée dite « haute », ladite enceinte comportant, à proximité de l'extrémité basse :
   - une entrée 110 primaire d'eau,
   - une entrée 115 de dihydrogène ou du gaz riche en dihydrogène et
   - une entrée 120 de dioxyde de carbone ou du gaz riche en dioxyde de carbone et
   à proximité de l'extrémité haute :
   - une sortie 125 pour méthane de synthèse ou pour un gaz riche en méthane de synthèse et
   - une sortie 130 primaire pour eau,
   - un matériau 135 support pour former un lit de flore méthanogène, présentant une densité inférieure à la densité de l'eau, mobile en translation le long de l'axe 101 longitudinal de l'enceinte, configuré pour recevoir une flore méthanogène et
   - entre le matériau support d'une part et la sortie pour méthane et la sortie pour eau d'autre part, une surface 140 de rétention du matériau support, perforée, et par exemple équipée de dispositifs crépines ou grilles laissant passer l'eau et les gaz mais retenant le matériau support, formant butée pour le déplacement longitudinal du matériau support au niveau de la position de ladite surface.

L'enceinte 105 est, par exemple, formée d'un volume fermé et étanche comportant des ouvertures pour positionner des entrées ou sorties de réactifs, de réactifs auxiliaires, de nutriments, d'eau ou de gaz dans le volume fermé. Ce volume fermé permet la constitution d'un milieu méthanogène à travers la réaction de méthanation s'y produisant.

La forme, interne et/ou externe, de l'enceinte 105 est sans importance pour la présente invention tant que l'enceinte est rendue étanche aux fuites. Préférentiellement, l'enceinte 105 présente une forme tubulaire, c'est-à-dire une forme cylindrique, pouvant être oblongue comme représenté en figures 1, 4 et 5.

Cette enceinte 105 présente une extrémité 107 longitudinale basse destinée à être positionnée à proximité du sol du lieu de positionnement du réacteur 100.

Cette enceinte 105 présente une extrémité 106 longitudinale haute destinée à être positionnée de manière distale du sol du lieu de positionnement du réacteur 100.

L'enceinte 105 comporte, à proximité de l'extrémité basse 107 :
- une entrée 110 primaire d'eau,
- une entrée 115 de dihydrogène ou d'un gaz riche en dihydrogène et
- une entrée 120 de dioxyde de carbone ou d'un gaz riche en dioxyde de carbone.

Chaque entrée, 110, 115 et 120, est, par exemple, une buse d'injection, une tuyère, un tube perforé, un réseau de tuyauterie équipé de crépines. Toutefois, tout organe d'injection de fluide habituellement utilisé dans un réacteur de méthanation biologique peut être utilisé pour réaliser chaque entrée, 110, 115 et 120.

Préférentiellement, chaque entrée, 110, 115 et 120, est positionnée de telle manière que, lorsque le matériau 135 support est en butée contre la surface 140 de rétention, chaque entrée, 110, 115 et 120, soit située dans l'eau sous le matériau 135 support.

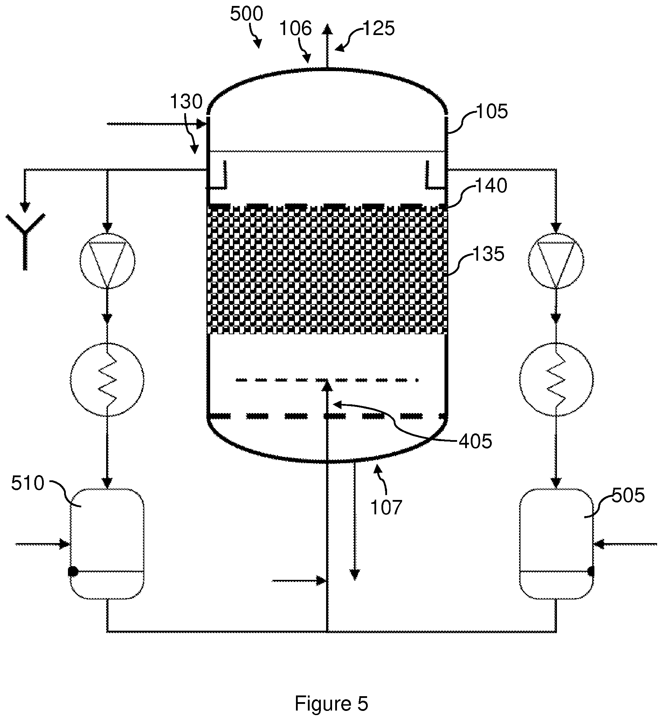

Dans des variantes, au moins deux entrées, 110, 115 et/ou 120, sont confondues. Lorsqu'au moins l'entrée 110 primaire pour eau et au moins une entrée parmi l'entrée 115 de dihydrogène et l'entrée 120 de dioxyde de carbone sont confondues, un moyen de dissolution de chaque gaz dont l'entrée, 115 et/ou 120, est confondu à l'entrée 110 primaire pour eau est positionné en amont de ladite entrée confondue. Un tel moyen de dissolution est décrit en regard de la figure 5.

Entre les entrées de gaz et d'eau de l'extrémité basse 107 est préférentiellement disposée une surface perforée 108 pouvant être équipée de grilles ou de crépines pour permettre une bonne répartition de l'eau traversant cette surface tout en évitant l'entrainement du matériau support.

L'enceinte 105 comporte, à proximité de l'extrémité haute 106 :
- une sortie 125 pour méthane de synthèse et
- une sortie 130 primaire pour eau.

Chaque sortie, 125 et 130, est, par exemple, une ouverture formée dans l'enceinte 105 reliée à une canalisation de transport.

Préférentiellement, la sortie 130 primaire pour eau est située plus à proximité de la surface de rétention 140 que la sortie 125 pour méthane de synthèse.

Dans des variantes la sortie 130 est équipée d'un dispositif (non représenté) de séparation pour éliminer les débris provenant du développement de microorganismes (cyclone, filtre par exemple). Ce dispositif peut être soit en amont de la sortie donc dans le réacteur 100, soit en aval de la sortie donc en dehors du réacteur 100.

Dans des variantes, le réacteur 100 comporte des tubes d'échange de chaleur immergés dans l'enceinte 105 et traversés par un fluide présentant une température compatible avec la température de fonctionnement nominale à l'intérieur de l'enceinte 105 lors du fonctionnement du réacteur 100. Le fluide peut être à une température plus élevée que l'intérieur de l'enceinte 105 pour permettre le réchauffage ou le maintien en température du réacteur 100, ou bien ce fluide peut être plus froid que l'intérieur de l'enceinte 105 pour permettre le maintien en température du réacteur 100 en évacuant un excès de chaleur.

Du fait de l'étanchéité de l'enceinte 105 et du positionnement des entrées et des sorties respectives, le dihydrogène et le dioxyde de carbone se déplacent verticalement dans l'enceinte 105, de l'extrémité basse vers l'extrémité haute, et traversent ainsi le matériau 135 support au cours de ce déplacement. Au cours de cette traversée, la flore méthanogène transforme ces réactifs en méthane, et le méthane, gazeux, se déplace également vers l'extrémité haute de l'enceinte 105, formant un ciel gazeux dans la partie supérieure de l'enceinte 105. Ce ciel gazeux est évacué par la sortie 125 pour méthane de synthèse.

L'eau entrée dans l'enceinte 105 remplit graduellement l'enceinte 105 et pousse le matériau 135 support, moins dense que l'eau, vers le haut de l'enceinte 105 jusqu'à ce que le matériau support 135 soit bloqué par la surface 140 de rétention. L'eau continue de remplir l'enceinte 105 jusqu'à atteindre la sortie 130 primaire pour eau, où l'eau est évacuée de l'enceinte 105. À l'eau injectée dans l'enceinte 105 s'ajoute l'eau formée par la réaction de méthanation.

Le matériau 135 support est, par exemple, composé de billes formée en un matériau moins dense que l'eau. Ces billes permettent l'accumulation de la flore méthanogène, cette flore étant formée par la famille de micro-organismes Archaea par exemple.

Les billes sont, par exemple, formées de charbon actif, polymères : polystyrène, polyacrylique et bien sûr les variantes sous forme de copolymères.

Ce matériau support 135 permet le développement d'une flore méthanogène concentrée au niveau du matériau support 135, ce matériau support 135 se déplaçant le long de l'enceinte 105 en fonction du remplissage de l'enceinte 105 en eau.

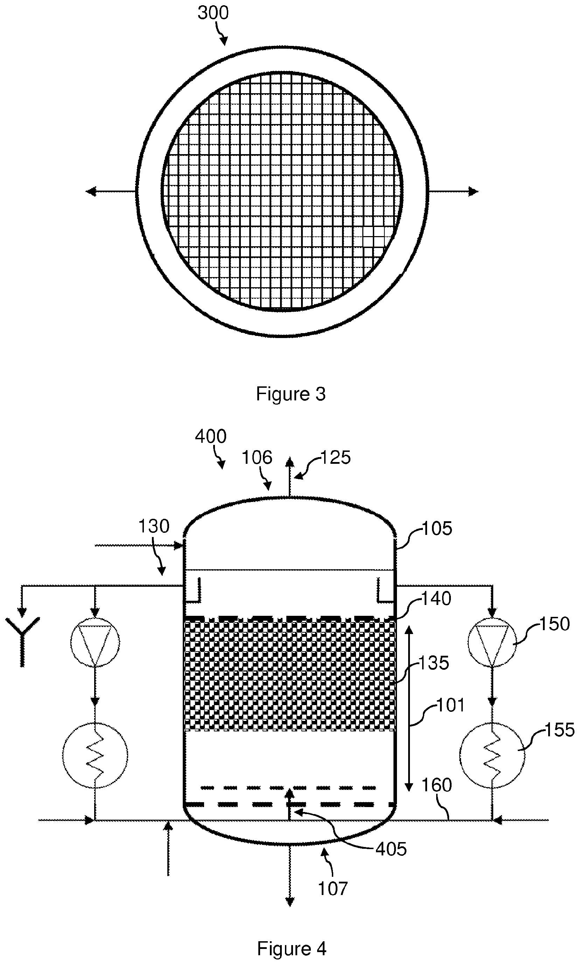

La surface 140 de rétention est, par exemple, un grillage ou une surface équipée de crépines. Un tel exemple de surface de rétention 140 est illustré, en figures 2 et 3.

Dans des variantes, la surface 140 de rétention est mobile en translation le long de l'axe longitudinal de l'enceinte, le mouvement de cette surface 140 étant limité par une butée positionnée en amont de la sortie 125 pour méthane et de la sortie 130 primaire pour eau le long de l'axe de déplacement longitudinal du matériau 135 support dans l'enceinte 105.

Ainsi, comme on le comprend, Le réacteur à lit flottant comme divulgué ci-dessus permet de créer une très grande surface de contact entre les micro-organismes et les gaz réactifs sans nécessiter d'agitateur mécanique. Ainsi il est possible de travailler à des pressions importantes sans risque de fuite de gaz et sans risque de panne d'un élément mécanique sensible que constitue un agitateur. Par ailleurs, l'utilisation d'un agitateur, pour assurer un bon contact entre les micro-organismes et les réactifs, nécessite de parfaitement maîtriser l'aéraulique du système. La présente invention permet de simplifier la conception du réacteur de méthanation biologique.

Dans des modes de réalisation, tel que celui représenté en figure 1, le réacteur 100 comporte une surverse 145 de collecte d'eau positionnée en amont de la sortie 125 pour méthane et de la sortie 130 primaire pour eau et en aval de la surface 140 de rétention le long de l'axe 101 de déplacement longitudinal du matériau 135 support dans l'enceinte 105, orientée vers l'extrémité 106 haute de l'enceinte, formant collecteur pour l'eau franchissant la surverse, la sortie 130 primaire pour eau étant positionnée dans ce collecteur.

La surverse 145 peut être une goulotte ou une rigole ou tout autre système de collecte d'eau gravitaire.

La surverse 145 est préférentiellement positionnée sur le pourtour intérieur de l'enceinte 105 de manière à longer au moins en partie l'intérieur du périmètre de la section transversale de l'enceinte 105. Cette surverse 145 permet de collecter l'excès d'eau dans l'enceinte 105.

Dans des modes de réalisation, tel que celui représenté en figure 1, le réacteur 100 comporte :
- au moins une pompe 150 de recirculation de l'eau traversant la sortie 130 pour eau de l'enceinte 105 et
- au moins un échangeur 155 thermique configuré pour chauffer ou refroidir l'eau sortie de l'enceinte,
l'eau recirculée, en sortie de l'échangeur thermique, étant au moins partiellement réinjectée dans l'enceinte à travers une conduite 160 d'injection.

Chaque pompe 150 est, par exemple, de type centrifuge, piston, à membrane, à vis, à engrenage ou péristaltique.

Chaque échangeur 155 est, par exemple, de type à faisceau tubulaire ou à plaque.

L'échangeur 155 thermique est contrôlé, par exemple, en fonction d'une valeur de température captée à l'intérieur de l'enceinte 105, au niveau du matériau 135 support, dans l'eau en-dessous ou au-dessus de la surface 140 de rétention. L'échangeur 155 thermique peut également être contrôlé en fonction de sa température de sortie.

Dans des variantes, le réacteur 100 comporte un capteur de température (non représenté) positionné à l'intérieur de l'enceinte 105 ou dans une conduite reliant l'échangeur 155 thermique et l'entrée 160 primaire pour eau recirculée.

Dans des variantes l'eau injectée dans le réacteur 100 est préchauffée ou pré-refroidie à une température déterminée, cette température pouvant dépendre d'une température captée par le capteur de température du réacteur 100 décrit ci-dessus.

L'eau injectée présente une température adaptée au maintien d'une température, dans l'enceinte, permettant le développement de la flore. Cette température est, par exemple, comprise entre 30 et 70°C et de préférence entre 60 à 65°C. L'eau est injectée, préférentiellement, à la température la plus basse possible, au-dessus de 0°C permettant le maintien de la température de développement de la flore compte tenu des conditions opératoires du réacteur.

L'eau ainsi recirculée est fournie à l'entrée 130 primaire pour eau à travers la conduite 160 d'injection.

Dans des modes de réalisation préférentiels, le réacteur 100 comporte un capteur 190 de niveau d'eau dans l'enceinte 105 et une évacuation 131 d'eau commandée par une vanne 195. Lorsque le niveau d'eau capté dans l'enceinte 105 est supérieur à une valeur limite déterminée, l'évacuation 131 est ouverte grâce la vanne 195, ce qui permet une sortie définitive d'eau en évitant toute recirculation liée à la pompe 150.

Dans des modes de réalisation, tel que celui représenté en figure 1, l'enceinte 105 comporte une entrée 165 pour nutriments et/ou réactifs additionnels et/ou d'eau.

Les nutriments favorisent le développement de la flore méthanogène tandis que les réactifs additionnels visent à limiter certains désagréments dans le milieu réactionnel à l'intérieur de l'enceinte 105. Ces réactifs additionnels comportent, par exemple, des régulateurs de pH ou des anti-mousses.

Dans des variantes, l'entrée 165 est confondue avec au moins l'une des entrées pour eau 110, pour dihydrogène 115 et/ou pour dioxyde de carbone 120.

L'injection de réactifs additionnels peut être réalisée en fonction de la capture de valeurs de grandeurs physiques à l'intérieur de l'enceinte 105. Par exemple, l'injection d'un réactif régulateur de pH peut être commandée en fonction d'une valeur de pH mesuré à l'intérieur de l'enceinte 105 ou de l'eau franchissant la sortie 130 primaire pour eau.

Dans des modes de réalisation, tel que celui représenté en figure 1, le réacteur 100 comporte, à proximité de l'extrémité 106 haute de l'enceinte 105, une entrée 170 secondaire pour eau et, à proximité de l'extrémité 107 basse de l'enceinte, une sortie 175 secondaire de reprise d'eau.

La sortie 175 secondaire de reprise d'eau est préférentiellement précédée d'un dispositif de répartition du débit d'eau, par exemple, un plancher perforé 108 tel que représenté dans la figure 1 ou un réseau de tuyauterie perforée.

L'eau ainsi injectée dans l'enceinte 105 circule par gravité sur le matériau 135 support et provoque le détachement d'agrégats de flore méthanogène qui sont entrainés vers l'extrémité basse de l'enceinte 105. De par la décohésion du lit, l'effet de la vitesse hydraulique autour des éléments du support et des chocs et des frottements entre les éléments du support, les matières en suspension potentielles et les excès de développement de flore méthanogène se détachent du support et sont entrainés avec l'eau vers la sortie 175 secondaire de reprise d'eau. Cette eau de lavage, ainsi que les effluents collectés sont évacués de l'enceinte 105 par la sortie secondaire 175 de reprise d'eau.

Dans des modes de réalisation, tel que celui représenté en figure 1, le réacteur 100 comporte une unité 180 de commande configurée pour commander l'actionnement d'un mode de fonctionnement de réacteur 100 parmi au moins deux, dans lequel :
- dans un mode actif :
   - l'entrée 170 secondaire pour eau et la sortie 175 secondaire de reprise d'eau sont désactivées et
   - l'entrée 110 primaire d'eau, l'entrée 115 de dihydrogène, l'entrée 120 de dioxyde de carbone et la sortie 130 primaire d'eau sont activées et
- dans un mode de lavage :
   - l'entrée 110 primaire d'eau, l'entrée 115 de dihydrogène, l'entrée 120 de dioxyde de carbone et la sortie 130 primaire d'eau sont désactivées et
   - l'entrée 170 secondaire pour eau et la sortie 175 secondaire de reprise d'eau sont activées.

Lorsque le réacteur comporte une entrée 165 pour nutriments et/ou pour réactifs additionnels, cette entrée 165 est désactivée dans le mode de lavage et activée dans le mode actif.

L'unité de commande 180 est, par exemple, un circuit électronique de commande exécutant un programme informatique configuré pour commander le passage du mode actif au mode de lavage, et inversement, selon un critère prédéterminé. Ce critère peut être temporel, le passage du mode actif au mode de lavage étant commandé au bout d'une durée déterminée de fonctionnement du réacteur 100 et, inversement, au bout d'une autre durée déterminée après le passage du réacteur 100 en mode de lavage.

Dans des modes de réalisation, tel que celui représenté en figure 1, le réacteur comporte un moyen 185 de mesure de perte de charge entre l'entrée et la sortie du réacteur représentative de la perte de charge dans le lit support, un changement de mode de fonctionnement étant commandé en fonction de la perte de charge mesurée.

Ce moyen 185 de mesure de perte de charge est, par exemple, un capteur différentiel de pression de part et d'autre du matériau support 135 ou par deux mesures de pression (non représentées).

Dans des variantes, le réacteur 100 comporte des tubes d'échange de chaleur immergés dans l'enceinte 105 et traversés par un fluide présentant une température compatible avec la température de fonctionnement nominale à l'intérieur de l'enceinte 105 lors du fonctionnement du réacteur 100. Le fluide peut être à une température plus élevée que l'intérieur de l'enceinte pour permettre le réchauffage ou le maintien en température du réacteur, ou bien il peut être plus froid que l'intérieur de l'enceinte pour permettre le maintien en température du réacteur en évacuant un excès de chaleur.

On observe sur la figure 2, schématiquement, une vue en coupe transversale de l'intérieur de l'enceinte 105 selon un plan positionné entre la surverse 145 et la sortie 125 pour méthane et orienté vers l'extrémité basse de l'enceinte 105. On observe, sur cette figure 2, une variante dans laquelle la surface 140 est équipée d'au moins une crépine de rétention du matériau support dimensionnée pour laisser passer le débit d'eau et de gaz et retenir le matériau support, la surverse 145 et au moins deux sorties 130 primaire et pour excès 131 d'eau situées dans la surverse 145.

On observe sur la figure 3, schématiquement, une vue en coupe transversale de l'intérieur de l'enceinte 105 selon un plan positionné entre la surverse 145 et la sortie 125 pour méthane et orienté vers l'extrémité basse de l'enceinte 105. On observe, sur cette figure 3, une variante dans laquelle la surface 140 est un grillage de rétention, la surverse 145 et au moins deux sorties 130 primaires et pour excès 131 d'eau situées dans la surverse 145.

On observe sur la figure 4, schématiquement, un mode de réalisation particulier du réacteur 400 objet de la présente invention. Ce réacteur 400 de méthanation biologique de dihydrogène ou de gaz riche en dihydrogène et de dioxyde de carbone ou de gaz riche en dioxyde de carbone, comporte :
- une enceinte 105 présentant une extrémité 107 longitudinale dite « basse » et une extrémité 106 longitudinale opposée dite « haute », ladite enceinte comportant, à proximité de l'extrémité basse, une entrée 405 confondue mais pouvant être distinctes :
   - d'eau,
   - de dihydrogène ou du gaz riche en dihydrogène et
   - de dioxyde de carbone ou du gaz riche en dioxyde de carbone et
   à proximité de l'extrémité haute :
   - une sortie 125 pour méthane de synthèse ou pour un gaz riche en méthane de synthèse et
   - une sortie 130 primaire pour eau,
- un matériau 135 support pour former un lit de flore méthanogène, présentant une densité inférieure à la densité de l'eau, mobile en translation le long de l'axe 101 longitudinal de l'enceinte, configuré pour recevoir une flore méthanogène et
- entre le matériau support d'une part et la sortie pour méthane et la sortie pour eau d'autre part, une surface 140 de rétention du matériau support, perforée, formant butée pour le déplacement longitudinal du matériau support au niveau de la position de ladite surface.

Dans ce mode de réalisation particulier, le réacteur 400 comporte au moins deux sorties 130 primaires pour eau et, pour chaque sortie, une pompe 150, chaque sortie peut être ou non équipées d'un échangeur 155 thermique, l'eau recirculée étant injectée dans une conduite commune d'injection mais pouvant être distinctes dans l'enceinte 105.

Dans ce mode de réalisation, l'eau recirculée est injectée dans un flux comportant au moins du dihydrogène et/ou du dioxyde de carbone, en amont de l'entrée 405 pour dihydrogène et/ou de l'entrée 405 pour dioxyde de carbone dans l'enceinte.

Dans ce mode de réalisation, les nutriments et/ou réactifs additionnels sont injectés dans la conduite d'injection en eau recirculée.

On observe sur la figure 5, schématiquement, un mode de réalisation particulier du réacteur 500 objet de la présente invention. Ce réacteur 500 de méthanation biologique de dihydrogène ou de gaz riche en dihydrogène et de dioxyde de carbone ou de gaz riche en dioxyde de carbone, comporte :
- une enceinte 105 présentant une extrémité 107 longitudinale dite « basse » et une extrémité 106 longitudinale opposée dite « haute », ladite enceinte comportant, à proximité de l'extrémité basse, une entrée 405 confondue mais pouvant être distinctes :
   - d'eau,
   - de dihydrogène ou du gaz riche en dihydrogène et
   - de dioxyde de carbone ou du gaz riche en dioxyde de carbone et
   à proximité de l'extrémité haute :
   - une sortie 125 pour méthane de synthèse ou pour gaz riche en méthane et
   - au moins une sortie 130 primaire pour eau,
- un matériau 135 support pour former un lit de flore méthanogène, présentant une densité inférieure à la densité de l'eau, mobile en translation le long de l'axe 101 longitudinal de l'enceinte, configuré pour recevoir une flore méthanogène et
- entre le matériau support d'une part et la sortie pour méthane et la sortie pour eau d'autre part, une surface 140 de rétention du matériau support, perforée, formant butée pour le déplacement longitudinal du matériau support au niveau de la position de ladite surface.

Dans ce mode de réalisation particulier, le réacteur 500 comporte au moins deux sorties 130 primaires pour eau et, pour chaque sortie, une pompe 150, chaque sortie peut être ou non équipées d'un échangeur 155 thermique.

Dans ce mode de réalisation particulier, le réacteur 500 comporte, en amont de l'entrée 405 confondue d'eau mais pouvant être distinctes, de dihydrogène et de dioxyde de carbone, un moyen 505 de dissolution du dioxyde de carbone ou du gaz riche en dioxyde de carbone et du dihydrogène ou gaz riche en dihydrogène dans l'eau.

Ce moyen 505 de dissolution est, par exemple, une colonne à garnissage, à pulvérisation, à bulle ou tout autre dispositif de dissolution afin de dissoudre un gaz dans un liquide.

Ce moyen 505 de dissolution peut être commun à la fois à une arrivée de dioxyde de carbone et à une arrivée de dihydrogène, ces arrivées étant éventuellement confondues.

Dans des modes de réalisation, tel que celui représenté en figure 5, le réacteur 500 comporte un moyen de dissolution 505 distinct pour le dioxyde de carbone ou le gaz riche en dioxyde de carbone d'une part et pour le dihydrogène ou le gaz riche en dihydrogène d'autre part.

L'eau recirculée est injectée au moins en partie dans le moyen 505 de dissolution si le réacteur 500 comporte un seul tel moyen 505 de dissolution. La fourniture d'eau recirculée au moyen 505 de dissolution dépend par exemple d'un niveau d'eau capté dans le moyen 505 de dissolution ou d'un débit de gaz entrant dans ledit moyen 505 de dissolution.

Si le réacteur 500 comporte un moyen, 505 et 510, de dissolution distinct pour chaque gaz, chaque moyen, 505 et 510, de dissolution peut être alimenté en eau recirculée issue d'une même sortie 130 primaire pour eau ou d'une sortie 130 primaire individuelle, l'eau recirculée étant distribuée entre les moyens, 505 et 510, de dissolution et éventuellement une conduite d'injection directe dans l'enceinte bipassant chaque moyen de dissolution.

Dans des modes de réalisation, tel que celui représenté en figure 5, le réacteur 500 comporte deux sorties 130 primaires pour eau et, pour chaque sortie 130, une pompe 150 et un échangeur thermique 155. L'eau recirculée par une première pompe 150 est injectée soit dans un premier moyen 505 de dissolution de dioxyde de carbone dans l'eau, soit directement dans l'enceinte 105. L'eau recirculée par une deuxième pompe 150 est injectée soit dans un deuxième moyen 510 de dissolution de dihydrogène dans l'eau, soit directement dans l'enceinte 105.

Dans ces modes de réalisation, le débit de recirculation d'eau vers chaque moyen de dissolution est commandé indépendamment. Chaque débit est commandé, par exemple, en fonction du débit de gaz entrant dans le moyen, 505 et 510, de dissolution.

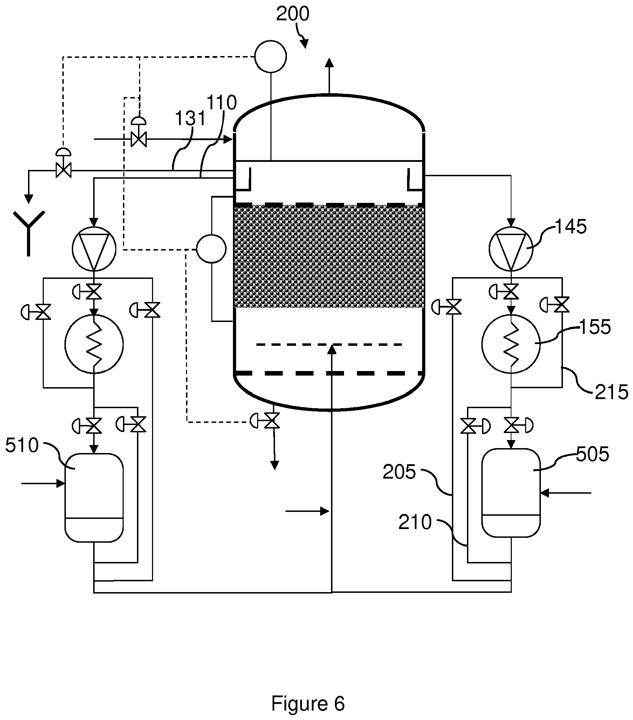

On observe sur la figure 6, en particulier, un mode de réalisation particulier du réacteur 200 identique au réacteur 100 tel que décrit en regard de la figure 1 qui comporte, de plus, au moins un bipasse, 205 et/ou 210 et/ou 215, d'au moins un élément parmi : un échangeur thermique 155 et/ou un moyen, 505 ou 510, de dissolution.

On observe, également en figure 5, que la sortie d'eau 131 d'évacuation et la sortie d'eau 110 primaire sont différenciées.

## Revendications

1. Réacteur (100, 200, 400, 500) de méthanation biologique de dihydrogène ou d'un gaz riche en dihydrogène et de dioxyde de carbone ou d'un gaz riche en dioxyde de carbone, **caractérisé en ce qu'**il comporte :
- une enceinte (105) présentant une extrémité (106) longitudinale dite « basse » et une extrémité (107) longitudinale opposée dite « haute », ladite enceinte comportant, à proximité de l'extrémité basse :
- une entrée (110, 405) primaire d'eau,
- une entrée (115, 405) de dihydrogène ou du gaz riche en dihydrogène et
- une entrée (120, 405) de dioxyde de carbone ou du gaz riche en dioxyde carbone et
à proximité de l'extrémité haute :
- une sortie (125) pour méthane de synthèse ou pour un gaz riche en méthane de synthèse et
- une sortie (130) primaire pour eau,
- un matériau (135) support pour former un lit de flore méthanogène, présentant une densité inférieure à la densité de l'eau, mobile en translation le long de l'axe (101) longitudinal de l'enceinte, configuré pour recevoir une flore méthanogène et
- entre le matériau support d'une part et la sortie pour méthane et la sortie pour eau d'autre part, une surface (140) de rétention du matériau support, perforée, formant butée pour le déplacement longitudinal du matériau support au niveau de la position de ladite surface.

2. Réacteur (100, 200, 400, 500) selon la revendication 1, qui comporte une surverse (145) de collecte d'eau positionnée en amont de la sortie (125) pour méthane et de la sortie (130) pour eau et en aval de la surface (140) de rétention le long de l'axe (101) de déplacement longitudinal du matériau (135) support dans l'enceinte (105), orientée vers l'extrémité (106) haute de l'enceinte, formant collecteur pour l'eau franchissant la surverse (145), la sortie primaire pour eau (130) étant positionnée dans ce collecteur.

3. Réacteur (100, 200, 400, 500) selon l'une des revendications 1 ou 2, qui comporte :
- au moins une pompe (150) de recirculation de l'eau traversant la sortie (130) pour eau de l'enceinte (105) et
- au moins un échangeur (155) thermique configuré pour chauffer ou refroidir l'eau sortie de l'enceinte,
l'eau recirculée, en sortie de l'échangeur thermique, étant au moins partiellement réinjectée dans l'enceinte à travers une conduite (160) d'injection.

4. Réacteur (400, 500) selon la revendication 3, dans lequel l'eau recirculée est injectée dans un flux comportant au moins du dihydrogène et/ou du dioxyde de carbone, en amont de l'entrée (405) pour dihydrogène et/ou de l'entrée (405) pour dioxyde de carbone dans l'enceinte.

5. Réacteur (100, 200, 400, 500) selon l'une des revendications 1 à 4, dans lequel l'enceinte (105) comporte une entrée (165) pour nutriments et/ou réactifs additionnels et/ou d'eau.

6. Réacteur (400, 500) selon la revendication 5 et l'une des revendications 3 ou 4, dans lequel les nutriments et/ou réactifs additionnels sont injectés dans la conduite d'injection en eau recirculée.

7. Réacteur (100, 200, 400, 500) selon l'une des revendications 1 à 6, qui comporte, à proximité de l'extrémité (106) haute de l'enceinte (105), une entrée (170) secondaire pour eau et, à proximité de l'extrémité (107) basse de l'enceinte, une sortie (175) secondaire de reprise d'eau.

8. Réacteur (100, 200, 400, 500) selon la revendication 7, qui comporte une unité (180) de commande configurée pour commander l'actionnement d'un mode de fonctionnement de réacteur parmi au moins deux, dans lequel :
- dans un mode actif :
- l'entrée (170) secondaire pour eau et la sortie (175) secondaire de reprise d'eau sont désactivées et
- l'entrée (110) primaire d'eau, l'entrée (115) de dihydrogène, l'entrée (120) de dioxyde de carbone et la sortie (130) primaire d'eau sont activées et
- dans un mode de lavage :
- l'entrée (110) primaire d'eau, l'entrée (115) de dihydrogène, l'entrée (120) de dioxyde de carbone et la sortie (130) primaire d'eau sont désactivées et
- l'entrée (170) secondaire pour eau et la sortie (175) secondaire de reprise d'eau sont activées.

9. Réacteur (100, 200, 400, 500) selon la revendication 8, qui comporte un moyen (185) de mesure de perte de charge entre l'entrée et la sortie du réacteur représentative de la perte de charge dans le lit support, un changement de mode de fonctionnement étant commandé en fonction de la perte de charge mesurée.

10. Réacteur (400, 500) selon l'une des revendications 1 à 9, dans lequel l'entrée pour dihydrogène ou gaz riche en dihydrogène, l'entrée pour dioxyde de carbone ou gaz riche en dioxyde de carbone et l'entrée primaire d'eau sont confondues (405).

11. Réacteur (200, 500) selon la revendication 10, qui comporte, en amont de l'entrée (405) confondue d'eau, de dihydrogène et de dioxyde de carbone, un moyen (505) de dissolution du dioxyde de carbone ou du gaz riche en dioxyde de carbone et du dihydrogène ou du gaz riche en dihydrogène dans l'eau.

12. Réacteur (200, 500) selon la revendication 11 et la revendication 3, dans lequel l'eau recirculée est injectée au moins en partie dans le moyen (505) de dissolution.

13. Réacteur (200, 500) selon la revendication 12, qui comporte un moyen (505) de dissolution du dioxyde de carbone ou gaz riche en dioxyde de carbone dans l'eau d'une part et un moyen (510) de dissolution du dihydrogène ou gaz riche en dihydrogène dans l'eau d'autre part, chaque moyen de dissolution étant alimenté en eau par de l'eau recirculée, le débit de recirculation d'eau vers chaque moyen de dissolution étant commandé indépendamment.

## Patentansprüche

1. Biologischer Methanisierungsreaktor (100, 200, 400, 500) für molekularen Wasserstoff oder ein an molekularem Wasserstoff reiches Gas und Kohlendioxid oder ein an Kohlendioxid reiches Gas, **dadurch gekennzeichnet, dass** es umfasst:
- eine Einfassung (105), die ein längliches, als "unteres"bezeichnetes Ende (106) und ein entgegengesetztes längliches, als "oberes" bezeichnetes Ende (107) aufweist, wobei die genannte Einfassung in der Nähe des unteren Endes aufweist:
- einen primären Wassereinlauf (110, 405),
- einen Einlauf (115, 405) für molekularen Wasserstoff oder an molekularem Wasserstoff reiches Gas und
- einen Einlauf (120, 405) für Kohlendioxid oder an Kohlendioxid reiches Gas und
in der Nähe des oberen Endes:
- einen Auslauf (125) für Synthesemethan oder für ein an Synthesemethan reiches Gas und
- einen primären Auslauf (130) für Wasser,
- ein Trägermaterial (135) zum Bilden eines Bettes aus methanbildender Flora, das eine geringere Dichte aufweist als die Dichte des Wassers, in Translation entlang der Längsachse (101) der Einfassung mobil, zum Aufnehmen einer methanbildenden Flora ausgestaltet ist und
- und zwischen dem Trägermaterial einerseits und dem Auslass für Methan und dem Auslass für Wasser andererseits eine Rückhaltefläche (140) des Trägermaterials, die perforiert ist und einen Anschlag für die längliche Verschiebung des Trägermaterials an der Position der genannten Fläche bildet.

2. Reaktor (100, 200, 400, 500) gemäß Anspruch 1, der einen Überlauf (145) zum Wasserauffangen umfasst, der dem Auslass (125) für Methan und dem Auslass (130) für Wasser vorgeschaltet und der Rückhaltefläche (140) entlang der Verschiebungslängsachse des Trägermaterials (135) in der Einfassung (105) nachgeschaltet ist, die zum oberen Ende (106) der Einfassung ausgerichtet ist und einen Auffang für das Wasser bildet, das zum Überlauf (145) übergeht, wobei der primäre Auslass für Wasser (130) in diesem Auffang angeordnet ist.

3. Reaktor (100, 200, 400, 500) gemäß einem der Ansprüche 1 oder 2, der umfasst:
- wenigstens eine Umwälzpumpe (150) des Wassers, das den Auslass (130) für das Wasser der Einfassung (105) durchquert und
- wenigstens einen Wärmetauscher (155), der zum Erhitzen oder Abkühlen des Auslasswassers der Einfassung ausgelegt ist,
wobei das umgewälzte Wasser am Auslass des Wärmetauschers wenigstens teilweise wieder durch eine Einspritzleitung (160) in die Einfassung eingespritzt ist.

4. Reaktor (400, 500) gemäß Anspruch 3, bei dem das umgewälzte Wasser in einen Fluss eingespritzt ist, der wenigstens molekularen Wasserstoff und/oder Kohlendioxid dem Einlass (405) für molekularen Wasserstoff und/oder dem Einlass (405) für Kohlendioxid in der Einfassung vorgeschaltet eingespritzt ist.

5. Reaktor (100, 200, 400, 500) gemäß einem der Ansprüche 1 bis 4, bei dem die Einfassung (105) einen Einlass (165) für Nährstoffe und/oder zusätzliche Reagenzien und/oder Wasser umfasst.

6. Reaktor (400, 500) gemäß Anspruch 5 und einem der Ansprüche 3 oder 4, bei dem die Nährstoffe und/oder zusätzlichen Reagenzien in die Einspritzleitung mit umgewälztem Wasser eingespritzt werden.

7. Reaktor (100, 200, 400, 500) gemäß einem der Ansprüche 1 bis 6, der in der Nähe des oberen Endes (106) der Einfassung (105) einen sekundären Einlass (170) für Wasser und in der Nähe des unteren Endes (107) der Einfassung einen sekundären Wasserentnahme-Auslass (175) umfasst.

8. Reaktor (100, 200, 400, 500) gemäß Anspruch 7, der eine Steuereinheit (180) umfasst, die zum Steuern der Betätigung eines Reaktor-Betriebsmodus aus mindestens zwei ausgelegt ist, bei der:
- in einem aktiven Modus:
- der sekundäre Einlass (170) für Wasser und der sekundäre Wasserentnahme-Auslass (175) deaktiviert sind und
- der primäre Wassereinlass (110), der Einlass (115) für molekularen Wasserstoff, der Einlass (120) für Kohlendioxid und der primäre Wasserauslass (130) aktiviert sind und
- in einem Waschmodus:
- der primäre Wassereinlass (110), der Einlass (115) für molekularen Wasserstoff, der Einlass (120) für Kohlendioxid und der primäre Wasserauslass (130) deaktiviert sind und
- der sekundäre Einlass (170) für Wasser und der sekundäre Wasserentnahme-Auslass (175) aktiviert sind.

9. Reaktor (100, 200, 400, 500) gemäß Anspruch 8, der ein Messmittel (185) des Lastverlustes zwischen dem Einlass und dem Auslass des Reaktors umfasst, das den Lastverlust in dem Trägerbett darstellt, wobei eine Änderung des Betriebsmodus in Abhängigkeit von dem gemessenen Lastverlust gesteuert ist.

10. Reaktor (400, 500) gemäß einem der Ansprüche 1 bis 9, bei dem der Einlass für molekularen Wasserstoff oder an molekularem Wasserstoff reichem Gas, der Einlass für Kohlendioxid oder an Kohlendioxid reichem Gas und der primäre Wassereinlass zusammenfallen (405).

11. Reaktor (200, 500) gemäß Anspruch 10, der dem zusammengefallenen Eingang (405) für Wasser, molekularen Wasserstoff und Kohlendioxid vorgeschaltet ist, ein Auflösungsmittel (505) des Kohlendioxids oder des an Kohlendioxid reichen Gases und des molekularen Wasserstoffs oder des an molekularen Wasserstoff reichen Gases im Wasser umfasst.

12. Reaktor (200, 500) gemäß Anspruch 11 und Anspruch 3, bei dem das umgewälzte Wasser wenigstens zum Teil in das Auflösungsmittel (505) eingespritzt ist.

13. Reaktor (200, 500) gemäß Anspruch 12, der ein Auflösungsmittel (505) des Kohlendioxids oder an Kohlendioxid reichen Gases im Wasser einerseits und ein Auflösungsmittel (510) des molekularen Wasserstoffs oder an molekularem Wasserstoff reichen Gases im Wasser andererseits umfasst, wobei jedes Auflösungsmittel durch das umgewälzte Wassers mit Wasser versorgt ist, wobei der Wasserumwälz-Durchsatz zu jedem Auflösungsmittel unabhängig gesteuert ist.

## Claims

1. Biological methanation reactor (100, 200, 400, 500) of hydrogen or hydrogen-rich gas, and of carbon dioxide or carbon dioxide-rich gas, **characterized in that** it comprises:
- a chamber (105) having one longitudinal extremity (106), referred to as "lower", and one opposite longitudinal extremity (107), referred to as "upper", said chamber comprising, in the vicinity of the lower extremity:
- a primary inlet (110, 405) for water,
- an inlet (115, 405) for hydrogen or hydrogen-rich gas, and
- an inlet (120, 405) for carbon dioxide or carbon dioxide-rich gas; and
in the vicinity of the upper extremity:
- an outlet (125) for synthetic methane or a gas rich in synthetic methane, and
- a primary outlet (130) for water;
- a substrate material (135) for forming a bed of methanogenic flora, having a density lower than the density of water, mobile in translation along the longitudinal axis (101) of the chamber, and configured to receive methanogenic flora; and
- between the substrate material on the one hand and the methane outlet and the water outlet on the other hand, a perforated surface (140) to retain the substrate material, forming a stop for the longitudinal movement of the substrate material at the position of said surface.

2. Reactor (100, 200, 400, 500) according to claim 1, which comprises a water collection overflow (145) positioned upstream from the methane outlet (125) and the water outlet (130) and downstream from the retention surface (140) along the axis (101) of longitudinal movement of the substrate material (135) in the chamber (105), oriented towards the upper extremity (106) of the chamber, forming a collector for the water passing over the overflow (145), the primary water outlet (130) being positioned in this collector.

3. Reactor (100, 200, 400, 500) according to one of claims 1 or 2, which comprises:
- at least one pump (150) for recirculating water passing through the water outlet (130) of the chamber (105); and
- at least one heat exchanger (155) configured to heat or cool the water exiting from the chamber;
the recirculated water, on output from the heat exchanger, being at least partially reinjected into the chamber through an injection pipe (160).

4. Reactor (400, 500) according to claim 3, wherein the recirculated water is injected into a flow comprising at least hydrogen and/or carbon dioxide, upstream from the hydrogen inlet (405) and/or the carbon dioxide inlet (405) in the chamber.

5. Reactor (100, 200, 400, 500) according to one of claims 1 to 4, wherein the chamber (105) comprises an inlet (165) for nutrients and/or additional reagents and/or water.

6. Reactor (400, 500) according to claim 5 and one of claims 3 or 4, wherein the nutrients and/or additional reagents are injected into the pipe for injecting recirculated water.

7. Reactor (100, 200, 400, 500) according to one of claims 1 to 6, which comprises, a secondary water inlet (170) in the vicinity of the upper extremity (106) of the chamber (105), and a secondary water recovery outlet (175) in the vicinity of the lower extremity (107) of the chamber.

8. Reactor (100, 200, 400, 500) according to claim 7, which comprises a control unit (180) configured to control the actuation of one reactor operating mode from among at least two, wherein:
- in an active mode:
- the secondary water inlet (170) and the secondary water recovery outlet (175) are deactivated, and
- the primary water inlet (110), the hydrogen inlet (115), the carbon dioxide inlet (120) and the primary water outlet (130) are activated; and
- in a cleaning mode:
- the primary water inlet (110), the hydrogen inlet (115), the carbon dioxide inlet (120) and the primary water outlet (130) are deactivated; and
- the secondary water inlet (170) and the secondary water recovery outlet (175) are activated.

9. Reactor (100, 200, 400, 500) according to claim 8, which comprises a means (185) for measuring the load loss between the reactor input and output, representative of the load loss in the substrate bed, a change in operating mode being controlled as a function of the load loss measured.

10. Reactor (400, 500) according to one of claims 1 to 9, wherein the inlet for hydrogen or hydrogen-rich gas, the inlet for carbon dioxide or carbon dioxide-rich gas, and the primary water inlet are one and the same (405).

11. Reactor (200, 500) according to claim 10, which comprises, upstream from the combined inlet (405) for water, hydrogen and carbon dioxide, a means (505) for dissolving carbon dioxide or carbon dioxide-rich gas, and hydrogen or hydrogen-rich gas in water.

12. Reactor (200, 500) according to claim 11 and claim 3, wherein at least part of the recirculated water is injected into the dissolution means (505).

13. Reactor (200, 500) according to claim 12, which comprises a means (505) for dissolving carbon dioxide or carbon dioxide-rich gas in water on the one hand and a means (510) for dissolving hydrogen or hydrogen-rich gas in water on the other hand, each dissolution means being supplied with water by means of the recirculated water, the water recirculation flow rate to each dissolution means being controlled independently.
